# EUROPEAN PATENT SPECIFICATION

(11) **EP 0 619 999 B1**
(45) Date of publication and mention of the grant of the patent: **10.01.2001**
(21) Application number: 94301882.0
(22) Date of filing: 16.03.1994
(51) Int. Cl.: A61K 7/42

(54) **Compositions containing sunscreens**
Sonnenschutzmittel
Compositions contenant des agents de protection solaire

(30) Priority: 14.04.1993 GB 9307658
(43) Date of publication of application: 19.10.1994
(73) Proprietor: TIOXIDE SPECIALTIES LIMITED, London W14 0QL (GB)
(72) Inventor: Dahms, Gerd Herbert, D-5620 Velbert 1 (DE)
(74) Representative: Graham, John George

(56) References cited:
- EP-A- 0 456 458
- EP-A- 0 559 319
- GB-A- 2 205 088
- GB-A- 2 226 018
- SEIFEN, ÖLE, FETTE, WACHSE, vol. 117, no. 10, 26 June 1991 AUGSBURG, DE, pages 397-398, 'Tioveil-ein neuer Sonnenschutzfilter'

## Description

The invention relates to compositions for application to human skin and particularly to compositions containing a combination of inorganic and organic sunscreens.

The use of inorganic oxides which have a particle size such that they are substantially transparent to visible light but reflect or absorb UV light to provide sunscreen compositions is known. However, in order to produce a sunscreen composition having a high sun protection factor (SPF) it is sometimes necessary to use relatively large amounts of such oxides and this can lead to undesirable visible tinting due to a small absorbance of visible light.

A combination of an inorganic oxide and an organic sunscreen can be used to obtain a high SPF with a relatively small amount of inorganic oxide but physiological damage to the body can occur following topical application of organic sunscreens in effective concentrations and consequently safety limits have been imposed on the quantity which is permitted in a composition for topical application.

It is therefore desirable to produce compositions which optimise the effectiveness of compounds used as UV absorbers and it is an object of this invention to provide a method of preparing compositions which improve the effectiveness of a combination of inorganic and organic UV absorbers. Some compositions having improved effectiveness are disclosed in EP-A-0 456 458.

According to the invention a process for the preparation of a composition suitable for topical application to human skin comprises mixing an aqueous dispersion of particles of a metallic oxide having an average primary particle size of less than 0.2 micrometre with one or more emulsifiers and an oil phase under conditions in which an oil-in-water emulsion is formed and with a hydrophobic organic sunscreen wherein the composition contains up to 10 per cent by weight metallic oxide and up to 7 per cent by weight hydrophobic organic sunscreen provided that said hydrophobic organic sunscreen does not comprise isoamyl p-methoxy cinnamate.

Compositions prepared according to the process of the invention have been shown to possess an SPF which is considerably greater than would be expected by calculating an SPF based on the additive effect of the metallic oxide and the organic sunscreen.

A particularly preferred embodiment of the process of the invention is based on the process described in the pending application filed in the United Kingdom under the application .number GB 9301515.4. According to this preferred embodiment a process for the preparation of a composition suitable for topical application to human skin comprises mixing an aqueous dispersion of particles of a metallic oxide having an average primary particle size of less than 0.2 micrometre with one or more emulsifiers and an oil phase under conditions in which an oil-in-water emulsion is formed and with a hydrophobic organic sunscreen wherein the total amount of emulsifiers present in the oil-in-water emulsion so formed is less than 10 per cent by weight, the particles of metallic oxide comprise up to 10 per cent by weight of the emulsion, the hydrophobic organic sunscreen comprises up to 7 per cent by weight of the emulsion, the oil phase comprises 5 per cent to 30 per cent by weight of the emulsion and an aqueous phase comprises at least 60 per cent by weight of the emulsion provided that said hydrophobic organic sunscreen does not comprise isoamyl p-methoxy cinnamate.

Preferably the metallic oxide used in the process of the invention comprises an oxide of titanium, zinc or iron and most preferably the metallic oxide is titanium dioxide.

The average primary particle size of the particles of metallic oxide used in the process of the invention is less than 0.2 micrometre and where the particles are substantially spherical then this size will be taken to represent the diameter. However, the invention also encompasses particles of metallic oxides which are non-spherical and in such cases the average primary particle size refers to the largest dimension.

Preferably the average primary particle size of the particles is from 0.01 to 0.15 micrometre and more preferably from 0.01 to 0.06 micrometre when they are substantially spherical. Particularly useful products can be prepared using substantially spherical particles having an average primary particle size in the range 0.01 to 0.03 micrometre. For particles having an acicular shape the average largest dimension of the primary particles is preferably less than 0.15 micrometre and more preferably from 0.02 to 0.10 micrometre.

When the metallic oxide is titanium dioxide the particles are preferably acicular in shape and have a ratio of largest dimension to smallest dimension of from 8:1 to 2:1.

When the metallic oxide is zinc oxide the particles preferably have an average primary particle size of 0.005 to 0.15 micrometre and more preferably have an average primary particle size of 0.03 to 0.07 micrometre.

The particles of metallic oxide may comprise substantially pure metallic oxide but may also carry an inorganic coating or organic coating. For example, particles of titanium dioxide can be coated with oxides of other elements such as oxides of aluminium, zirconium or silicon and a form of acicular, coated titanium dioxide which is especially useful in the process of this invention is disclosed in UK Patent GB 2 205 088.

The particles of metallic oxides may also carry, if desired, a coating of one or more organic materials such as polyols, amines, alkanolamines, polymeric organic silicon compounds, hydrophilic polymers such as polyacrylamide, polyacrylic acid, carboxymethyl cellulose and xanthan gum or surfactants.

The metallic oxide is used in the process of the invention in a quantity sufficient to ensure a concentration of up to 10 weight per cent with respect to the final emulsion. When the metallic oxide is titanium dioxide, it is preferably present in an amount of from 1 to 6 per cent by weight and most preferably it is present in an amount of from 4 to 5 per cent by weight. When the metallic oxide is zinc oxide, the preferred amount is from 3 to 8 per cent by weight and the most preferred amount is from 5 to 7 per cent by weight.

In carrying out the process of the invention an aqueous dispersion of a metallic oxide having a primary particle size as hereinbefore defined is used. Typically, the dispersion is prepared by milling the metallic oxide in water in the presence of a particulate grinding medium and in the presence of a dispersing agent.

UK Patent Application GB 2 226 018 discloses an aqueous dispersion of titanium dioxide containing a dispersing agent which is a polycarboxylic acid or a salt thereof and in which the titanium dioxide has an acicular shape. The dispersions described in GB 2 226 018 are particularly suitable for use in the method of the current invention when it is desired to produce an oil-in-water emulsion containing titanium dioxide.

The technique described in GB 2 226 018 can be used to prepare aqueous dispersions of metallic oxides other than titanium dioxide which are suitable for use in the process of the invention.

Suitable dispersing agents which can be used to prepare dispersions of metallic oxides according to GB 2 226 018 include polyacrylic acids, substituted acrylic acid polymers, acrylic copolymers, sodium and/or ammonium salts of polyacrylic acids and sodium and/or ammonium salts of acrylic copolymers.

GB 2 260 018 discloses aqueous dispersions containing from 20 to 60 per cent by weight titanium dioxide and concentrations of titanium dioxide or other metallic oxides in this range are suitable for the practice of this invention. Preferably, the concentration of metallic oxide in the aqueous dispersion is from 30 to 50 weight per cent with respect to weight of dispersion.

The hydrophobic organic sunscreens which are of use in the current invention are organic compounds which have been shown to be useful when added to compositions for the purpose of absorbing UV light and which are substantially insoluble in water at about 20°C. Such compounds include p-methoxy cinnamic acid esters other than isoamyl p-methoxy cinnamate, salicylic acid esters, p-amino benzoic acid esters, non-sulphonated benzophenone derivatives, derivatives of dibenzoyl methane and esters of 2-cyanoacrylic acid. Specific examples of useful organic sunscreens are given in the following table, identified by their INCI name (formerly CTFA name) and, in some cases, other common names.

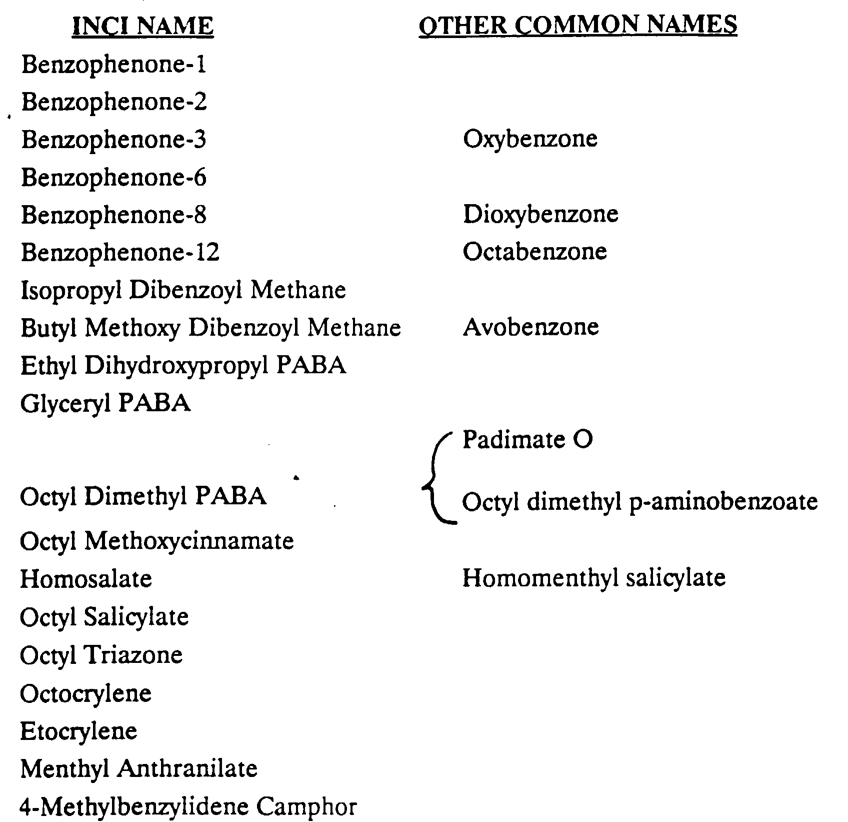

The preferred organic sunscreen is octyl methoxycinnamate.

A mixture of two or more hydrophobic organic sunscreens can be used.

The quantity of hydrophobic organic sunscreens used will depend to some extent upon the nature of the organic sunscreen but is up to 7 per cent by weight based on weight of emulsion. Preferably the amount of hydrophobic organic sunscreen is from 1 to 6 per cent by weight. When the hydrophobic organic sunscreen is octyl methoxycinnamate the preferred quantity is from 1 to 4 per cent by weight.

In the particularly preferred embodiment of the process of the current invention the emulsions produced contain a relatively small amount of one or more emulsifiers by comparison with previously known emulsions. Usually, the total amount of emulsifiers used in the emulsion is less than 7 per cent by weight and, preferably, less than 5 per cent by weight. Also the quantity of hydrophilic emulsifiers used is preferably less than 10 per cent by weight of the total quantity of emulsifiers used and, in the most preferred case, no hydrophilic emulsifiers are used.

Generally, in this preferred embodiment, the hydrophile-lipophile balance (HLB value) of the emulsifier system used is less than 6 and, preferably, the HLB value is less than 5.

When hydrophilic emulsifiers are used then suitable emulsifiers include polyoxyethylene derivatives of sorbitan fatty acid esters, polyoxyethylene fatty acid esters, polyoxyethylene fatty ethers, phosphate esters, fatty alcohol sulphates, polyglycoside ethers, polyglycoside esters and alkali metal salts of sulphosuccinate esters.

Hydrophobic emulsifiers suitable for use in the preferred embodiment of the process of the invention include lipid emulsifiers such as fatty alcohols, fatty acids, glyceryl esters, sorbitan esters, methylglycoside esters and sucrose esters. Generally, in contrast to hydrophilic emulsifiers these emulsifiers are easy to produce from renewable raw materials, are readily biodegradable and do not contain toxic side products.

The components of the oil phase used in the process of the invention are oleophilic, cosmetically acceptable compounds. Examples of suitable compounds include paraffin oils, silicone oils, triglyceride esters and esters of fatty acids and fatty alcohols.

The aqueous dispersion of metallic oxide, emulsifier and oil phase are mixed under conditions which produce an oil-in-water emulsion.

In a typical process, the aqueous dispersion is mixed, if required, with other water miscible ingredients to form an aqueous phase and this phase and the oil phase are separately heated to at least 40°C, preferably to at least 60°C and more preferably to at least 70°C. These two phases are then mixed under vigorous stirring in the presence of the emulsifier or emulsifiers. Mixing equipment which has found use for preparing cosmetic creams, lotions etc. is suitable for preparing the emulsions. High shear mixers/homogenisers are particularly suitable.

The emulsifier(s) are usually added to the aqueous phase containing the aqueous dispersion before the oil phase is mixed with the aqueous phase.

The hydrophobic organic sunscreen is preferably added to the oil phase before this is mixed with the aqueous dispersion but it can also be mixed with the emulsion after this has been prepared from the aqueous dispersion of metallic oxide and oil phase.

Other ingredients can be added to the emulsion and these ingredients may be introduced in any convenient manner. For example they can be mixed with the emulsion or added to the aqueous dispersion or the oil phase before these components are mixed together. As examples, perfumes, antioxidants, moisturisers, thickeners and preservatives are normally added to emulsions to produce a commercially acceptable cosmetic product.

The oil-in-water emulsions produced according to the process of this invention find use as sunscreen compositions, as skin protectants, as moisturisers and as after-sun lotions and generally have the property of being transparent to visible light although they absorb or scatter UV light.

The measured SPF for an emulsion prepared according to the process of the invention is considerably higher than expected. For example, an emulsion containing 4 per cent titanium dioxide by weight and no organic sunscreen was shown to have an SPF of 11. Previous experience indicates that the addition of 1 per cent octyl methoxycinnamate by weight to such a composition should increase the SPF to between 12 and 13. In fact, an emulsion containing a combination of 4 per cent titanium dioxide and 1 per cent octyl methoxycinnamate by weight prepared according to the method of the invention has been found to have an SPF of 16.

The invention is further illustrated by the following Examples.

### EXAMPLE 1

An oil-in-water emulsion suitable for use as a sunscreen was prepared according to the following formulation:-

| | | % by weight |
|---|---|---|
| 1) | Isopropyl Myristate | 4.00 |
| 2) | Paraffin oil | 6.50 |
| 3) | Grape seed oil | 2.50 |
| 4) | Stearyl alcohol | 2.00 |
| 5) | Petrolatum | 2.00 |
| 6) | Octyl Methoxycinnamate (sold under the Trade Name Parsol MCX) | 1.00 |
| 7) | Sorbitan Stearate (sold under the Trade Name Span 60) | 6.00 |
| 8) | Disodium Ricinoleamido MEA-Sulfosuccinate (sold under the Trade Name Rewoderm S1333) | 0.20 |
| 9) | Dispersion of 40% by weight titanium dioxide in water (sold under the Trade Name Tioveil AQ) | 10.00 |
| 10) | Glycerol | 4.00 |
| 11) | Allantoin | 0.20 |
| 12) | D-Panthenol | 0.80 |
| 13) | Demineralised water | 60.58 |
| 14) | Mixture of alkyl parabens in phenoxyethanol (sold under the Trade Name Phenonip) | 0.02 |
| 15) | Perfume | 0.20 |

Ingredients 1 to 6 were mixed to form an oil phase and heated to 80°C.

Ingredients 7 to 13 were mixed to form an aqueous phase and heated to 80°C.

The oil phase was added to the aqueous phase with high-speed stirring (Braun kitchen mixer type 4169). High-speed stirring was continued for one minute to homogenize the mixture. The resulting emulsion was cooled to 25°C in a water bath, with slow agitation. Ingredients 14 and 15 were then added with moderate stirring.

The product so formed had an in-vitro sun protection factor (SPF) of 16.1 (measured by the method of Diffey and Robson : J. Soc. Cosmet. Chem. 40, p.127-133 (1989)).

A product made according to the same composition, but omitting the octyl methoxycinnamate, had an in-vitro SPF of 11.2.

### EXAMPLE 2

An oil-in-water emulsion was prepared according to the method of Example 1 except that the amount of octyl methoxycinnamate was increased to 3% by weight and the amount of water was reduced to 58.58% to compensate.

Using the same technique as in Example 1, the in-vitro SPF was found to be 26.3.

### EXAMPLE 3

Oil-in-water emulsions were prepared according to the following formulations:-

| | | **A** | **B** | **C** |
|---|---|---|---|---|
| | | % | % | % |
| | | by weight | by weight | by weight |
| 1) | Isopropyl Myristate | 4.00 | 4.00 | 4.00 |
| 2) | Paraffin oil | 6.50 | 6.50 | 6.50 |
| 3) | Grape seed oil | 2.50 | 2.50 | 2.50 |
| 4) | Stearyl alcohol | 2.00 | 2.00 | 2.00 |
| 5) | Petrolatum | 2.00 | 2.00 | 2.00 |
| 6) | Octyl Triazone (sold under the Trade Name Uvinul T150) | - | 3.00 | 3.00 |
| 7) | Sorbitan Stearate (sold under the Trade Name Montane 60) | 6.00 | 6.00 | 6.00 |
| 8) | Disodium Ricinoleamido MEA-Sulfosuccinate (sold under the Trade Name Rewoderm S1333) | 0.20 | 0.20 | 0.20 |
| 9) | Dispersion of 40% by weight titanium dioxide in water (sold under the Trade Name Tioveil AQ) | 10.00 | - | 10.00 |
| 10) | Glycerol | 4.00 | 4.00 | 4.00 |
| 11) | Allantoin | 0.20 | 0.20 | 0.20 |
| 12) | D-Panthenol | 0.80 | 0.80 | 0.80 |
| 13) | Demineralised water | 61.40 | 68.40 | 58.40 |
| 14) | Mixture of alkyl parabens in phenoxyethanol (sold under the Trade Name Phenonip) | 0.20 | 0.20 | 0.20 |
| 15) | Perfume | 0.20 | 0.20 | 0.20 |

Ingredients 1 to 6 were mixed to form an oil phase and heated to 80°C.

Ingredients 7 to 13 were mixed to form an aqueous phase and heated to 80°C.

The oil phase was added to the aqueous phase with high speed stirring (Braun mixer type 4169). High speed stirring was continued for one minute to homogenise the mixture. The resulting emulsion was cooled to 25°C in a water bath, with slow agitation. Ingredients 14 and 15 were then added with moderate stirring.

The product A, containing only titanium dioxide as active sunscreen agent, had an in-vitro SPF of 6.8. The product B, containing only octyl triazone as a sunscreen agent, had an in-vitro SPF of 1.5. The product C, containing both active sunscreen agents, had an in-vitro SPF of 14.8.

### EXAMPLE 4

Oil-in-water emulsions were prepared according to the following formulations:-

| | | **D** | **E** | **F** |
|---|---|---|---|---|
| | | % | % | % |
| | | by weight | by weight | by weight |
| 1) | Isopropyl Myristate | 4.00 | 4.00 | 4.00 |
| 2) | Paraffin oil | 6.50 | 6.50 | 6.50 |
| 3) | Grape seed oil | 2.50 | 2.50 | 2.50 |
| 4) | Stearyl alcohol | 2.00 | 2.00 | 2.00 |
| 5) | Petrolatum | 2.00 | 2.00 | 2.00 |
| 6) | Octyl Salicylate (sold under the Trade Name Neo Heliopan OS) | - | 3.00 | 3.00 |
| 7) | Sorbitan Stearate (sold under the Trade Name Montane 60) | 6.00 | 6.00 | 6.00 |
| 8) | Disodium Ricinoleamido MEA-Sulfosuccinate (sold under the Trade Name Rewoderm S1333) | 0.20 | 0.20 | 0.20 |
| 9) | Dispersion of 40% by weight titanium dioxide in water (sold under the Trade Name Tioveil | 10.00 AQ) | - | 10.00 |
| 10) | Glycerol | 4.00 | 4.00 | 4.00 |
| 11) | Allantoin | 0.20 | 0.20 | 0.20 |
| 12) | D-Panthenol | 0.80 | 0.80 | 0.80 |
| 13) | Demineralised water | 61.40 | 68.40 | 58.40 |
| 14) | Mixture of alkyl parabens in phenoxyethanol (sold under the Trade Name Phenonip) | 0.20 | 0.20 | 0.20 |
| 15) | Perfume | 0.20 | 0.20 | 0.20 |

Ingredients 1 to 6 were mixed to form an oil phase and heated to 80°C.

Ingredients 7 to 13 were mixed to form an aqueous phase and heated to 80°C.

The oil phase was added to the aqueous phase with high speed stirring (Braun mixer type 4169). High speed stirring was continued for one minute to homogenise the mixture. The resulting emulsion was cooled to 25°C in a water bath, with slow agitation. Ingredients 14 and 15 were then added with moderate stirring.

The product D, containing only titanium dioxide as active sunscreen agent, had an in-vitro SPF of 6.8. The product E, containing only octyl salicylate as active sunscreen agent, had an in-vitro SPF of 2.5. The product F, containing both active sunscreen agents, had an in-vitro SPF of 10.1.

### EXAMPLE 5

Oil-in-water emulsions were prepared according to the following formulations:

| | | **G** | **H** | **I** |
|---|---|---|---|---|
| | | % | % | % |
| | | by weight | by weight | by weight |
| 1) | Isopropyl Myristate | 4.00 | 4.00 | 4.00 |
| 2) | Paraffin oil | 6.50 | 6.50 | 6.50 |
| 3) | Grape seed oil | 2.50 | 2.50 | 2.50 |
| 4) | Stearyl alcohol | 2.00 | 2.00 | 2.00 |
| 5) | Petrolatum | 2.00 | 2.00 | 2.00 |
| 6) | Octyl Dimethyl PABA (sold under the Trade Name Escalol 507) | - | 3.00 | 3.00 |
| 7) | Sorbitan Stearate (sold under the Trade Name Span 60) | 6.00 | 6.00 | 6.00 |
| 8) | Disodium Ricinoleamido MEA-Sulfosuccinate (sold under the Trade Name Rewoderm S1333) | 0.20 | 0.20 | 0.20 |
| 9) | Dispersion of 40% by weight titanium dioxide in water (sold under the Trade Name Tioveil AQ) | 10.00 | - | 10.00 |
| 10) | Glycerol | 4.00 | 4.00 | 4.00 |
| 11) | Allantoin | 0.20 | 0.20 | 0.20 |
| 12) | D-Panthenol | 0.80 | 0.80 | 0.80 |
| 13) | Demineralised water | 61.40 | 68.40 | 58.40 |
| 14) | Mixture of alkyl parabens in phenoxyethanol (sold under the Trade Name Phenonip) | 0.20 | 0.20 | 0.20 |
| 15) | Perfume | 0.20 | 0.20 | 0.20 |

Ingredients 1 to 6 were mixed to form an oil phase and heated to 80°C.

Ingredients 7 to 13 were mixed to form an aqueous phase and heated to 80°C.

The oil phase was added to the aqueous phase with high speed stirring (Braun mixer type 4169). High speed stirring was continued for one minute to homogenise the mixture. The resulting emulsion was cooled to 25°C in a water bath, with slow agitation. Ingredients 14 and 15 were then added with moderate stirring.

The product G, containing only titanium dioxide as active sunscreen agent, had an in-vitro SPF of 6.5. The product H, containing only octyl dimethyl PABA as active sunscreen agent, had an in-vitro SPF of 4.7. The product I, containing both active sunscreen agents, had an in-vitro SPF of 16.2.

## Claims

1. A process for the preparation of a composition suitable for topical application to human skin characterised in that an aqueous dispersion of particles of a metallic oxide having an average primary particle size less than 0.2 micrometre is mixed with one or more emulsifiers and an oil phase under conditions in which an oil-in-water emulsion is formed and with a hydrophobic sunscreen and the composition contains up to 10 per cent by weight metallic oxide and up to 7 per cent by weight hydrophobic organic sunscreen provided that said hydrophobic organic sunscreen does not comprise isoamyl p-methoxy cinnamate.

2. A process according to claim 1 characterised in that the total amount of emulsifiers present in the oil-in-water emulsion is less than 10 per cent by weight, the oil phase comprises 5 per cent to 30 per cent by weight of the emulsion and an aqueous phase comprises at least 60 per cent by weight of the emulsion.

3. A process according to claim 1 or 2 characterised in that the metallic oxide is an oxide of titanium, zinc or iron.

4. A process according to any one of the preceding claims characterised in that the particles of metallic oxide are substantially spherical and have an average primary particle size in the range 0.01 to 0.15 micrometre.

5. A process according to any one of claims 1 to 3 characterised in that the particles of metallic oxide are acicular and the average largest dimension of the primary particles is from 0.02 to 0.15 micrometre.

6. A process according to any one of claims 1 to 3 or 5 characterised in that the metallic oxide is titanium dioxide and the particles are acicular and have a ratio of largest dimension to smallest dimension in the range 8:1 to 2:1.

7. A process according to any one of claims 1 to 5 characterised in that the metallic oxide is zinc oxide and the particles thereof have an average primary particle size in the range 0.005 to 0.15 micrometre.

8. A process according to any one of claims 1 to 6 characterised in that the metallic oxide is titanium dioxide and the particles thereof carry an inorganic coating comprising an oxide of aluminium, zirconium or silicon.

9. A process according to any one of the preceding claims characterised in that the particles of metallic oxide carry a coating of a polyol, an amine, an alkanolamine, a polymeric silicon compound, a hydrophilic polymer or a surfactant.

10. A process according to any one of claims 1 to 6, 8 or 9 characterised in that the metallic oxide is titanium dioxide and is present in an amount of from 1 to 6 per cent by weight with respect to total weight of emulsion.

11. A process according to any one of claims 1 to 5, 7 or 9 characterised in that the metallic oxide is zinc oxide and is present in an amount of from 3 to 8 per cent by weight with respect to total weight of emulsion.

12. A process according to any one of the preceding claims characterised in that the hydrophobic organic sunscreen is a p-methoxy cinnamic acid ester other than isoamyl p-methoxy cinnamate, a salicylic acid ester, a p-aminobenzoic acid ester, a non-sulphonated benzophenone derivative, a derivative of dibenzoyl methane or an ester of 2-cyanoacrylic acid.

13. A process according to any one of the preceding claims characterised in that the hydrophobic organic sunscreen is present in an amount in the range 1 to 6 per cent by weight based on total weight of emulsion.

14. A process according to any one of the preceding claims characterised in that the hydrophobic organic sunscreen is octyl methoxy cinnamate and is present in an amount in the range 1 to 4 per cent by weight based on total weight of emulsion.

15. A process according to any one of claims 2 to 14 characterised in that the total amount of emulsifiers present in the emulsion is less than 7 per cent by weight.

16. A process according to any one of claims 2 to 15 characterised in that less than 10 per cent of the total weight of emulsifiers is hydrophilic emulsifier.

17. A process according to any one of claims 2 to 16 characterised in that the hydrophile-lipophile balance of the emulsifier system present is less than 6.

18. A process according to any one of the preceding claims characterised in that the aqueous dispersion of metallic oxide is prepared by milling the metallic oxide in water in the presence of a particulate grinding medium and in the presence of a dispersing agent comprising a polycarboxylic acid or a salt thereof.

19. A process according to any one of the preceding claims characterised in that the aqueous dispersion of metallic oxide contains from 20 to 60 per cent by weight metallic oxide.

20. A process according to any one of the preceding claims characterised in that the aqueous dispersion and oil phase are separately heated to a temperature of at least 40°C and the dispersion and oil phase are subsequently mixed under vigorous stirring in the presence of the one or more emulsifiers.

## Patentansprüche

1. Verfahren zur Herstellung einer zur topischen Anwendung auf die menschliche Haut geeigneten Zusammensetzung, dadurch gekennzeichnet, dass eine wässrige Dispersion von Partikeln eines Metalloxids mit einer durchschnittlichen primären Partikelgröße von wenigers als 0,2 µm mit einem oder mehreren Emulgatoren und einer Ölphase unter Bedingungen, bei denen eine Öl-in-Wasser-Emulsion gebildet wird, und mit einem hydrophoben Sonnenschutzmittel vermischt wird und wobei die Zusammensetzung bis zu 10 Gew.% Metalloxid und bis zu 7 Gew.% eines hydrophoben organischen Sonnenchutzmittels enthält, mit der Maßgabe, dass der hydrophobe organische Sonnenschutzmittel nicht Isoamyl-p-methoxyzinnamat umfasst.

2. Verfahren gemäss Anspruch 1, dadurch gekennzeichnet, dass die Gesamtmenge der in der Öl-in-Wasser-Emulsion vorhandenen Emulgatoren weniger als 10 Gew.% beträgt, die Ölphase 5 bis 30 Gew.% der Emulsion umfasst und eine wässrige Phase wenigstens 60 Gew.% der Emulsion umfasst.

3. Verfahren gemäss Anspruch 1 oder 2, dadurch gekenngeichnet, dass das Metalloxid ein Oxid von Titan, Zink oder eisen ist.

4. Verfahren gemäss einem der vorhergehenden Ansprüche, dadurch gekennzeichnet, dass die Partikel des Metalloxids im wesentlichen sphärisch sind und eine durchschnittliche primäre Partikelgröße in dem Bereich von 0,01 bis 0,15 µm aufweisen.

5. Verfahren gemäss einem der Ansprüche 1 bis 3, dadurch gekennzeichnet, dass die Partikel des Metalloxids nadelförmig sind und die durchschnittliche größte Dimension der primären Partikel 0,02 bis 0,15 µm beträgt.

6. Verfahren gemäss einem der Ansprüche 1 bis 3 oder 5, dadurch gekennzeichnet, dass das Metalloxid Titandioxid ist und die Partikel nadelförmig sind und ein Verhältnis der größten Dimension zu der kleinsten Dimension in dem Bereich von 8 : 1 bis 2 : 1 haben.

7. Verfahren gemäss einem der Ansprüche 1 bis 5, dadurch gekennzeichnet, dass das Metalloxid Zinkoxid ist und die Partikel hiervon eine durchschnittliche primäre Partikelgröße in dem Bereich von 0,005 bis 0,15 µm haben.

8. Verfahren gemäss einem der Ansprüche 1 bis 6, dadurch gekennzeichnet, dass das Metalloxid Titandioxid ist und die Partikel hiervon eine anorganische Beschichtung tragen, die ein Oxid des Aluminiums, Zirkoniums oder Silizium umfasst.

9. Verfahren gemäss einem der vorhergehenden Ansprüche, dadurch gekennzeichnet, dass die Partikel des Metalloxids eine Beschichtung mit einem Polyol, einem Amin, einem Alkanolamin, einer polymeren Siliziumverbindung, einem hydrophilen Polymer oder einem oberflächenaktiven Wirkstoff tragen.

10. Verfahren gemäss einem der Ansprüche 1 bis 6, 8 oder 9, dadurch gekennzeichnet, dass das Metalloxid Titandioxid ist und in einer Menge von 1 bis 6 Gew.% im Hinblick des Gesamtgewichts der Emulsion vorhanden ist.

11. Verfahren gemäss einem der Ansprüche 1 bis 5, 7 oder 9, dadurch gekennzeichnet, dass das Metalloxid Zinkoxid ist und in einer Menge von 3 bis 8 Gew.% im Bezug auf das Gesamt gewicht der Emulsion vorhanden ist.

12. Verfahren gemäss einem der vorhergehenden Ansprüche, dadurch gekennzeichnet, dass der hydrophobe organische Sonnenschutzmittel ein von Isoamyl-p-Methoxyzinnamat verschiedener p-Methoxyzimtsäureester, ein Salicylsäureester, ein p-Aminobenzoesäureester, ein nicht sulfoniertes Benzophenonderivat, ein Derivat des Dibenzoylmethans oder ein Ester der 2-Cyanoacrylsäure ist.

13. Verfahren gemäss einem der vorhergehenden Ansprüche, dadurch gekennzeichnet, dass der hydrophobe organische Sonnenschutzmittel in einer Menge im Bereich von 1 bis 6 Gew.%, basierend auf dem Gesamtgewicht der Emulsion, vorhanden ist.

14. Verfahren gemäss einem der vorhergehenden Ansprüche, dadurch gekennzeichnet, dass der hydrophobe organische Sonnenschutzmittel Oktylmethoxyzinnamat ist und in einer Menge im Bereich von 1 bis 4 Gew.%, basierend auf dem Gesamtgewicht der Emulsion, vorhanden ist.

15. Verfahren gemäss einem der Ansprüche 2 bis 14, dadurch gekennzeichnet, dass die Gesamtmenge der in der Emulsion vorhandenen Emulgatoren weniger als 7 Gew.% beträgt.

16. Verfahren gemäss einem der Ansprüche 2 bis 15, dadurch gekennzeichnet, dass weniger als 10 % des Gesamtgewichts der Emulgatoren ein hydrophiler Emulgator ist.

17. Verfahren gemäss einem der Ansprüche 2 bis 16, dadurch gekennzeichnet, dass die hydrophile-lipophile Balance des vorhandenen Emulgatorsystems wenigers als 6 beträgt.

18. Verfahren gemäss einem der vorhergehenden Ansprüche, dadurch gekennzeichnet, dass die wässrige Dispersion des Metalloxids durch Mahlen des Metalloxids in Wasser in Gegenwart eines aus Teilchen bestehenden Mahlmediums und in Gegenwart eines Dispergierungsmittels, das eine Polycarboxysäure oder ein Salz hiervon umfasst, hergestellt wird.

19. Verfahren gemäss einem der vorhergehenden Ansprüche, dadurch gekennzeichnet, dass die wässrige Dispersion des Metalloxids 20 - 60 Gew.-% Metalloxid enthält.

20. Verfahren gemäss einem der vorhergehenden Ansprüche, dadurch gekennzeichnet, dass die wässrige Dispersion und die Ölphase voneinander getrennt bis auf eine Temperatur von wenigstens 40°C erhitzt werden und anschließend die Dispersion und die Ölphase unter heftigem Rühren in Gegenwart eines oder mehrerer Emulgatoren vermischt werden.

## Revendications

1. Procédé pour la préparation d'une composition appropriée à l'application topique sur la peau humaine, caractérisé en ce qu'une dispersion aqueuse de particules d'un oxyde métallique ayant une taille moyenne de particules primaires de moins de 0,2 micromètre est mélangée avec un ou plusieurs émulsifiants et avec une phase huileuse dans des conditions dans lesquelles il se forme une émulsion huile-dans-l'eau et avec un agent de protection solaire hydrophobe, et en ce que la composition contient jusqu'à 10 pour cent en poids d'oxyde métallique et jusqu'à 7 pour cent en poids d'agent de protection solaire organique hydrophobe, à la condition que ledit agent de protection solaire organique hydrophobe ne comprenne pas de p-méthoxycinnamate d'isoamyle.

2. Procédé selon la revendication 1, caractérisé en ce que la quantité totale des émulsifiants présents dans l'émulsion huile-dans-l'eau est inférieure à 10 pour cent en poids, que la phase huileuse constitue 5 pour cent à 30 pour cent du poids de l'émulsion et que la phase aqueuse constitue au moins 60 pour cent en poids de l'émulsion.

3. Procédé selon la revendication 1 ou la revendication 2, caractérisé en ce que l'oxyde métallique est un oxyde de titane, de zinc ou de fer.

4. Procédé selon l'une quelconque des revendications précédentes, caractérisé en ce que les particules d'oxyde métallique sont pratiquement sphériques et ont une taille moyenne de particules primaires comprise entre 0,01 et 0,15 micromètre.

5. Procédé selon l'une quelconque des revendications 1 à 3, caractérisé en ce que les particules d'oxyde métallique sont aciculaires et que la plus grande dimension moyenne des particules primaires est comprise entre 0,02 et 0,15 micromètres.

6. Procédé selon l'une quelconque des revendications 1 à 3 ou 5, caractérisé en ce que l'oxyde métallique est le dioxyde de titane et que les particules sont aciculaires et ont un rapport (plus grande dimension)/(plus petite dimension) compris entre 8/1 et 2/1.

7. Procédé selon l'une quelconque des revendications 1 à 5, caractérisé en ce que l'oxyde métallique est l'oxyde de zinc et que les particules de celui-ci ont une taille moyenne de particules primaires comprise entre 0,005 et 0,15 micromètre.

8. Procédé selon l'une quelconque des revendications 1 à 6, caractérisé en ce que l'oxyde métallique est le dioxyde de titane et que les particules de celui-ci portent un revêtement inorganique comprenant un oxyde d'aluminium, de zirconium ou de silicium.

9. Procédé selon l'une quelconque des revendications précédentes, caractérisé en ce que les particules d'oxyde métallique sont revêtues d'un polyol, d'une amine, d'une alcanolamine, d'un composé polymère de silicium, d'un polymère hydrophile ou d'un surfactant.

10. Procédé selon l'une quelconque des revendications 1 à 6, 8 ou 9, caractérisé en ce que l'oxyde métallique est le dioxyde de titane et qu'il est présent en quantité comprise entre 1 et 6 pour cent en poids par rapport au poids total de l'émulsion.

11. Procédé selon l'une quelconque des revendications 1 à 5, 7 ou 9, caractérisé en ce que l'oxyde métallique est l'oxyde de zinc et qu'il est présent en quantité comprise entre 3 et 8 pour cent en poids par rapport au poids total de l'émulsion.

12. Procédé selon l'une quelconque des revendications précédentes, caractérisé en ce que l'agent de protection solaire organique hydrophobe est un ester de l'acide p-méthoxycinnamique autre que le p-méthoxycinnamate d'isoamyle, un ester de l'acide salicylique, un ester de l'acide p-aminobenzoïque, un dérivé benzophénone non sulfoné, un dérivé du dibenzoylméthane ou un ester de l'acide 2-cyanoacrylique.

13. Procédé selon l'une quelconque des revendications précédentes, caractérisé en ce que l'agent de protection solaire organique hydrophobe est présent en quantité comprise entre 1 et 6 pour cent en poids par rapport au poids total de l'émulsion.

14. Procédé selon l'une quelconque des revendications précédentes, caractérisé en ce que l'agent de protection solaire organique hydrophobe est le méthoxycinnamate d'octyle et qu'il est présent en quantité comprise entre 1 et 4 pour cent par rapport au poids total de l'émulsion.

15. Procédé selon l'une quelconque des revendications 2 à 14, caractérisé en ce que la quantité totale des émulsifiants présents dans l'émulsion est inférieure à 7 pour cent en poids.

16. Procédé selon l'une quelconque des revendications 2 à 15, caractérisé en ce que moins de 10 pour cent du poids total des émulsifiants sont constitués d'émulsifiant hydrophile.

17. Procédé selon l'une quelconque des revendications 2 à 16, caractérisé en ce que la balance hydrophile-lipophile du système émulsifiant présent est inférieur e à 6.

18. Procédé selon l'une quelconque des revendications précédentes, caractérisé en ce que la dispersion aqueuse d'oxyde métallique est préparée par broyage de l'oxyde métallique dans l'eau en présence d'un milieu particulaire de broyage et en présence d'un agent dispersant comprenant un acide polycarboxylique ou un sel de celui-ci.

19. Procédé selon l'une quelconque des revendications précédentes, caractérisé en ce que la dispersion aqueuse d'oxyde métallique contient de 20 à 60 pour cent en poids d'oxyde métallique.

20. Procédé selon l'une quelconque des revendications précédentes, caractérisé en ce que la dispersion aqueuse et la phase huileuse sont chauffées séparément à une température d'au moins 40°C et que la dispersion et la phase huileuse sont ultérieurement mélangées sous forte agitation en présence d'un ou de plusieurs émulsifiants.
